# EUROPÄISCHE PATENTSCHRIFT

(11) **EP 1 687 407 B1**
(45) Veröffentlichungstag und Bekanntmachung des Hinweises auf die Patenterteilung: **14.01.2009**
(21) Anmeldenummer: 04819251.2
(22) Anmeldetag: 24.11.2004
(51) Int. Cl.: C12P 7/26

(54) **FERMENTATION EINWERTIGER SEKUNDÄRER ALKOHOLE ZU ENTSPRECHENDEN KETONEN**
FERMENTATION OF MONOVALENT SECONDARY ALCOHOLS IN ORDER TO FORM CORRESPONDING KETONES
FERMENTATION D'ALCOOLS MONOVALENTS SECONDAIRES EN CÉTONES CORRESPONDANTES

(30) Priorität: 28.11.2003 DE 10355734
(43) Veröffentlichungstag der Anmeldung: 09.08.2006
(73) Patentinhaber: Symrise GmbH & Co. KG, 37603 Holzminden (DE)
(72) Erfinder: RABENHORST, Jürgen, 37671 Höxter (DE)
(74) Vertreter: Eisenführ, Speiser & Partner
(86) Internationale Anmeldenummer: PCT/EP2004/053076
(87) Internationale Veröffentlichungsnummer: WO 2005/052173

(56) Entgegenhaltungen:
- EP-A- 1 081 229
- MOLINARI, F. ET AL.: "Microbial Biotransformations to obtain (R)- and (S)-2-octanol" ANNALI DI MICROBIOLOGIA ED ENZIMOLOGIA, Bd. 47, Nr. 1, 1997, Seiten 131-137, XP008046383
- DATABASE BIOTECHABS 1994, "Alcohol oxidation using immobilized microorganism; and aldehyde, ketone or carboxylic acid preparation using immobilized yeast, bacterium or fungus e.g. 2-octanone production using Nocardia farcinica and Rhodococcus equi" XP002326766 Database accession no. 1994-05878 -& JP 06 000090 A (KANSAI PAINT CO LTD) 11. Januar 1994 (1994-01-11)
- MOONMANGMEE, D. ET AL.: "Purification and Characterization of Membrane-bound Quinoprotein Cyclic Alcohol Dehydrogenase from Gluconobacter frateurii CHM 9" BIOSCIENCE, BIOTECHNOLOGY, AND BIOCHEMISTRY, Bd. 65, Nr. 12, Dezember 2001 (2001-12), Seiten 2763-2772, XP008046372
- GUPTA, A. ET AL.: "Gluconobacter oxydans: Its Biotechological Applications" JOURNAL OF MOLECULAR MICROBIOLOGY AND BIOTECHNOLOGY, Bd. 3, Nr. 3, Juli 2001 (2001-07), Seiten 445-456, XP008026573

## Beschreibung

Die vorliegende Erfindung betrifft ein Fermentationsverfahren zum Umsetzen eines einwertigen sekundären Alkohols mit 5 oder mehr Kohlenstoffatomen zu dem entsprechenden Keton, dadurch gekennzeichnet, daß Pentan-2-on aus 2-Pentanol, Heptan-2-on aus2-Heptanol, 1-Penten-3-on aus 1-Penten-3-ol, 1-Hexen-3-on aus 1-Hexen-3-ol, Hexan-3-on aus 3-Hexanol, Heptan-3-on aus 3-Heptanol hergestellt wird.

Aus der Literatur ist die Oxidation primärer Alkohole zu den entsprechenden Säuren bekannt, wie beispielsweise die Veröffentlichungen EP 289 822, DE 195 03 598, J. Chem. Tech. Biotechnol. 1997, 68, 214 - 218, und J. Chem. Tech. Biotechnol. 1997, 70, 294 - 298 belegen. In Lett. Appl. Microbiol. 1995, 20, 365 - 368 wird die Umsetzung von verschiedenen Alkoholen zu Säuren und zu 2-Butanon beschrieben und auf die stärker inhibierende Wirkung von 2-Butanon im Vergleich zu den Säuren hingewiesen. Zudem ist die asymmetrische Reduktion von Ketonen zur Herstellung von enantiomerenreinen Alkoholen mit Gluconobacter oxydans bekannt (Adlerkreuz, Enzyme Microb. Technol. 1991, 13, 9-14). Die Oxidation von sekundären Alkoholen ist mit Alkoholoxidasen aus verschiedenen Kohlenwasserstoff abbauenden Hefen bekannt (Appl. Microbiol. Biotechnol. 1992, 37, 66 - 73; Tetrahedron Asymmetry 2000, 11, 2367 - 2373). Diese Reaktion verläuft mit hoher Stereoselektivität, so dass nur maximal 50 Prozent des Substrats umgesetzt werden, was die mögliche Produktausbeute limitiert. Darüber hinaus wird NAD als Cofaktor benötigt. NAD muss daher entweder in equimolaren Mengen zugegeben werden, was aus wirtschaftlichen Gründen nicht möglich ist, oder mittels einer zweiten enzymatischen Umsetzung das entstandene NADH regeneriert werden, was ebenfalls mit Aufwand und Kosten verbunden ist. Schließlich beschreiben Adachi et al., Appl. Microbiol Biotechnol. (2003) 60:643-653, die Oxidation mehrwertiger Alkohole zu Molekülen mit gleichzeitig Hydroxy- und Ketofunktionen.

Das japanische Patent JP 06000090 (Veröffentlichungsdatum: 11.01.1994) offenbart ein Verfahren zur Umsetzung von 2-Octanol zu Octan-2-on unter Verwendung eines Bakteriums der Gattung *Gluconobacter.* Die europäische Patentanmeldung EP 1 081 229 A (Veröffentlichungsdatum 07.03.2001) offenbart die Verwendung des Bakterienstammes *Gluconobacter* sp. DSM 12884 zur Umsetzung von primären Alkoholen zu den entsprechenden Carbonsäuren.

Die bisherigen Fermentationsverfahren erreichen oft nur unerwünscht niedrige Produktausbeuten. Enzymatische Verfahren, wie beispielsweise von Adlerkreuz (a.a.O.) beschrieben, erfordern zudem die Regeneration von zur Herstellung der Ketone notwendigen Coenzymen, was die Durchführung der enzymatischen Verfahren erschwert und verteuert.

Aufgabe der vorliegenden Erfindung war es daher, ein Verfahren anzugeben, mit dessen Hilfe einwertige sekundäre Alkohole mit 5 oder mehr Kohlenstoffatomen in die entsprechenden Ketone umgesetzt werden können. Das Verfahren sollte möglichst einfach handhabbar sein, gute Produktausbeuten in geringer Reaktionszeit ermöglichen und kostengünstig durchführbar sein. Zudem sollen Mittel zum Durchführen dieser Verfahren angegeben werden. Insbesondere sollte das anzugebende Verfahren sowie die zu seiner

Durchführung geeigneten Mittel das Umsetzen eines aliphatischen einwertigen sekundären Alkohols zum entsprechenden Keton ermöglichen.

Es wird deshalb ein Verfahren zum Umsetzen eines einwertigen sekundären Alkohols mit 5 oder mehr Kohlenstoffatomen zu dem entsprechenden Keton angegeben, wobei das Verfahren das Fermentieren des Alkohols zum Keton mit Hilfe eines Bakteriums der Gattung Gluconobacter und/oder Acetobacter in einem Fermentationsmedium umfasst.

Mit Hilfe dieses Verfahrens lassen sich die angestrebten Vorteile erreichen. Insbesondere gelingt das Umsetzen eines geradkettigen einwertigen sekundären Alkohols zum entsprechenden Keton leicht, in guter Ausbeute, reproduzierbar und kostengünstig. Zudem können gleichzeitig mehrere der einwertigen sekundären Alkohole zu den entsprechenden Ketonen fermentiert werden.

Besonders vorteilhaft wirkt sich aus, dass die Umsetzung des Alkohols zum Keton in einem Fermentationsverfahren, also unter Verwendung ganzer Zellen des zum Fermentieren verwendeten Bakteriums, durchgeführt wird. Beste Ergebnisse konnten dabei erzielt werden, wenn das Fermentieren mit Hilfe eines vermehrungsfähigen Bakteriums der genannten Gattungen erfolgt. Dabei gilt ein Bakterium dann als vermehrungsfähig, wenn sich seine Zellzahl nach Überführen in ein übliches Kultivierungsmedium (z.B. DSM Medium 105: Glucose 100 g; Hefeextrakt 10 g; CaCO₃ 20.0 g; destilliertes Wasser 1000 ml eingestellt auf pH 6.8) und Kultivieren unter üblichen Kultivierungsbedingungen (z.B. 25 °C) innerhalb von 24 Stunden verdoppelt, wobei gegebenenfalls eine Mindest-Zellkonzentration zu Beginn des Kultivierens eingehalten werden muss. Als besonders vorteilhaft erweist sich dabei, dass auf die Regeneration von Coenzymen und Kofaktoren verzichtet werden kann, was die Verfahrensdurchführung erheblich vereinfacht. Es wird ein einwertiger sekundärer Alkohol mit 5 oder mehr Kohlenstoffatomen, höchstens jedoch 20 und besonders bevorzugt höchstens 14 Kohlenstoffatomen zu dem entsprechenden Keton mit Hilfe eines Bakteriums der Gattung Gluconobacter fermentiert. Die nachfolgend angegebenen besonders bevorzugten Ausführungsformen betreffen insbesondere auch das Fermentieren einwertiger sekundärer Alkohole mit den soeben angegebenen bevorzugten höchsten Anzahlen an Kohlenstoffatomen.

Vorzugsweise erfolgt die Fermentation eines einwertigen sekundären Alkohols mit 5 oder mehr Kohlenstoffatomen (vorzugsweise mit höchstens 20 Kohlenstoffatomen und besonders bevorzugt mit höchstens 14 Kohlenstoffatomen) mit Hilfe eines Bakteriums der Gattung Gluconobacter, wobei besonders gute Produktausbeuten vor allem bei Fermentationen mit Hilfe von Bakterien der Spezies Gluconobacter oxydans erreicht werden. Besonders gute Ergebnisse können erzielt werden, wenn das Fermentieren mit Hilfe eines Bakteriums des Stammes Gluconobacter oxydans ssp. suboxydans DSM 12884 bewirkt wird. Die Verwendung dieses Stammes zum Fermentieren eines einwertigen sekundären Alkohols, insbesondere eines aliphatischen und vorzugsweise geradkettigen Alkohols, mit 5 oder mehr Kohlenstoffatomen, bevorzugt höchstens 20 Kohlenstoffatomen und besonders bevorzugt höchstens 14 Kohlenstoffatomen, zum entsprechenden Keton ist daher besonders bevorzugt. Dieser Stamm ist in der Lage, mindestens 1 g/l 1-Penten-3-on innerhalb von 48 Stunden bei Fermentation in DSM-Medium 105 enthaltend 3 g/l 1-Penten-3-ol bei 25 °C zu fermentieren.

Zweckmäßigerweise wird das Fermentieren durchgeführt mit einer Reinkultur der genannten Bakterien, insbesondere von Gluconobacter sp. DSM 12884.

Als Nährmedium für die erfindungsgemäß eingesetzten Organismen kommen synthetische, halbsynthetische oder komplexe Medien in Betracht. Die Nährmedien können insbesondere als Fermentationsmedium verwendet werden und können kohlenstoffhaltige und stickstoffhaltige Verbindungen, anorganische Salze, gegebenenfalls Spurenelemente sowie Vitamine enthalten.

Als kohlenstoffhaltige Verbindungen können Kohlenhydrate, Kohlenwasserstoffe oder organische Grundchemikalien verwendet werden. Beispiele für vorzugsweise verwendbare Verbindungen sind Zucker, Alkohole bzw. Zuckeralkohole, organische Säuren oder komplexe Gemische. Ein bevorzugter Zuckeralkohol ist Mannit.

Als organische Säuren können vorzugsweise Zitronensäure oder Essigsäure verwendet werden. Als Bestandteile halbsynthetischer oder komplexer Medien sind insbesondere Malzextrakt, Hefeextrakt, Casein oder Caseinhydrolysat bevorzugt.

Als stickstoffhaltige Substrate können insbesondere anorganische Verbindungen verwendet werden, beispielsweise Nitrate und Ammoniumsalze. Ebenso können organische Stickstoffquellen verwendet werden wie Hefeextrakt, Sojamehl, Baumwollsaatmehl, Weizengluten, Casein, Caseinhydrolysat und Maisquellwasser.

Zu den einsetzbaren anorganischen Salzen zählen beispielsweise Sulfate, Nitrate, Chloride, Carbonate und Phosphate. Als Metalle enthalten die genannte Salze vorzugsweise Natrium, Kalium, Magnesium, Mangan, Calcium, Zink und/oder Eisen.

Bevorzugt ist es ferner, vor dem Fermentieren eine Kultur des zum Fermentieren verwendeten Bakteriums in einem Kultivierungsmedium vorzukultivieren, das Mannit, Malzextrakt, Hefeextrakt, Sojamehl, Baumwollsaatmehl, Weizengluten, Casein, Caseinhydrolysat, Maisquellwasser, Zitronensäure, Essigsäure oder Mischungen zweier oder mehrerer dieser Bestandteile enthält und bei Beginn des Vorkultivierens einen pH-Wert von 4 bis 8 hat. Das Kultivieren erleichtert das Fermentieren insbesondere in dem Fall, in dem Bakterien verwendet werden sollen, die sich vor Beginn des Fermentierens in einem Ruhezustand befinden, insbesondere in einer Glycerinkultur. Es ist ebenfalls möglich, zunächst in einem Kultivierungsmedium die zum Fermentieren verwendeten Bakterien vorzukultivieren und nach dem Vorkultivieren zu diesem Medium den oder die zu fermentierenden Alkohol(e) hinzuzufügen. Besonders bewährt als Kultivierungsmedium hat sich ein Medium bestehend aus 1-2 g D-Mannit und 1-2 g Hefeextrakt, bezogen auf 100 ml Medium, bei einem pH-Wert von 5-6.

Die Temperatur für das Fermentieren und das Kultivieren liegt vorzugsweise im Bereich von 10 bis 40 °C. Besonders bevorzugt ist der Bereich von 20 bis 35 °C, höchst bevorzugt sind 25 bis 27 °C, da bei diesen Temperaturen besonders gute Umsätze und Produktausbeuten erzielt werden konnten.

Der pH-Wert des Fermentations- und des Kultivierungsmediums beträgt zu Beginn des Fermentierens bzw. zu Beginn des Kultivierens bevorzugt 4 bis 8, wobei der Bereich von 4,8 bis 6,3 besonders bevorzugt ist.

Besonders gute Fermentationsergebnisse konnten erhalten werden, wenn zum Zeitpunkt der Substratzugabe die optische Dichte der Kultur bei mindestens OD₆₀₀ 2,0, die Lebendkeimzahl mindestens 1 x 10⁸/ml und die Konzentration gelösten Sauerstoffs im Fermentationsmedium vor der Substratzugabe nicht mehr als 10 % beträgt.

Grundsätzlich können für die Durchführung des erfindungsgemäßen Verfahrens alle dem Fachmann bekannten Bioreaktoren eingesetzt werden. Vorzugsweise wird ein zur Durchführung von Submersverfahren geeigneter Bioreaktor zum Fermentieren verwendet. Das heißt, es können erfindungsgemäß Bioreaktoren ohne oder mit mechanischer Mischeinrichtung eingesetzt werden. Zu ersteren zählen z.B. Schüttelapparaturen, Blasensäulen- oder Schlaufenreaktoren. Zu letzteren gehören Bioreaktoren mit Rührern in beliebiger Gestaltung.

Das erfindungsgemäße Verfahren kann kontinuierlich oder diskontinuierlich durchgeführt werden. Die Dauer der Fermentation bis zum Erreichen einer maximalen Produktmenge hängt von der speziellen Art des eingesetzten Organismus ab. Besonders gute Produktausbeuten konnten bei einer Fermentation von 2 bis zu 200 Stunden erreicht werden, wobei die Fermentation vorzugsweise 8 bis 78 Stunden lang durchgeführt wird.

Mit dem erfindungsgemäßen Verfahren können insbesondere folgende Ketone hergestellt werden: Pentan-2-on, Heptan-2-on, 1-Penten-3-on, 1-Hexen-3-on, Hexan-3-on, Heptan-3-on.

Die Erfindung wird anhand der nachfolgenden Beispiele näher erläutert:

### Beispiel 1 - Herstellen der Vorkultur

Ein 500 ml Erlenmeyerkolben mit seitlichem Einstich mit 100 ml sterilem Kulturmedium, bestehend aus 1,25 g D-Mannit und 1,25 g Hefeextrakt bei pH 5,6, wird mit 0,9 ml einer Glycerinkulur von Gluconobacter sp. DSM 12884 angeimpft. Der Kolben wird 16 Stunden lang auf einer rotierenden Schüttelmaschine bei 25 °C und 100 Upm inkubiert.

### Beispiel 2 - Herstellung von natürlichem 1-Penten-3-on aus 1-Penten-3-ol

Es werden 125 g Mannit und 125 g Hefeextrakt in 9,9 L Wasser in einem 10l Fermenter gelöst, 10 ml Antischaummittel hinzu gegeben und der pH-Wert auf 6,3 eingestellt. Das so hergestellte Fermentationsmedium wir 30 Minuten lang bei 121 °C sterilisiert. Nach dem Abkühlen auf 25 °C wird der Fermenter mit der Vorkultur aus Beispiel 1 angeimpft.

Während der Umsetzung von 1-Penten-3-ol und einer dieser voraufgehenden Wachstumsphase wird das Fermentationsmedium gerührt. Die Drehzahl des Rührers beträgt 500 Upm, die Zuluft 5 NUmin; die Temperatur 25 °C. Nach Einstellen dieser Parameter wird das Fermentationsmedium mit der 100 ml Vorkultur gemäß Beispiel 1 angeimpft.

Nach ca. 23 Stunden Kultivierungszeit (Wachstumsphase) wird das Substrat 1-Penten-3-ol (150 ml) über einen Trichter dem Fermenter zugeführt und die Fermentation begonnen. Zeitgleich wird die Zuluft von 5 NUmin auf 1 NUmin (≙ 0,1 vvm) gedrosselt. Der pH-Wert bleibt während der Kultivierungszeit und der Fermentation relativ stabil.

Da sowohl das Substrat 1-Penten-3-ol als auch das Produkt 1-Penten-3-on sehr leicht flüchtig sind, wird die Abluft des Fermenters über einen Aktivkühler geleitet und anschließend in einer Kühlfalle aufgefangen, die mit einem Isopropanol-Trockeneis-Gemisch gekühlt wird.

Die Fermentation wird nach ca. 70 Stunden beendet. Die Endkonzentration des 1-Penten-3-ons beträgt im Fermentationsmedium laut HPLC ca. 5 g/l. Das Substrat 1-Penten-3-ol liegt noch in einer Konzentration von etwa 2 g/l im Fermentationsmedium vor. In der Kühlfalle befinden sich am Ende des Prozesses ca. 20 g 1-Penten-3-on.

### Beispiel 3 - Gewinnung von 2-Pentanon aus 2-Pentanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 200 µl 2-Pentanol zugegeben, die Kolben werden mit sterilen Schliffstopfen verschlossen und weiter inkubiert. 24 und 48 Stunden nach Zugabe des 2-Pentanols wird je 1 ml Probe entnommen, mit 2 ml Hexan extrahiert und gaschromatographisch analysiert. Nach 24 Stunden wurden 83 % (Flächenprozent) 2-Pentanon und 10,5 % 2-Pentanol gefunden. Nach 48 Stunden liegt der Gehalt an 2-Pentanon bei über 92 %.

### Beispiel 4 - Gewinnung von 2-Heptanon aus 2-Heptanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 100 µl 2-Heptanol zugegeben, die Kolben mit sterilen Schliffstopfen verschlossen und weiter inkubiert. 24 und 48 Stunden nach Zugabe des 2-Heptanol wird je 1 ml Probe entnommen, mit 2 ml Hexan extrahiert und gaschromatographisch analysiert. Nach 24 Stunden wurden 54,7 % (Flächenprozent) 2-Heptanon und 35 % 2-Heptanol gefunden. Nach 48 Stunden liegt der Gehalt an 2-Heptanon bei über 59 %, während noch 31,9 % 2-Heptanol vorliegen.

### Beispiel 5 - Gewinnung von 2-Octanon aus 2-Octanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 100 µl 2-Octanol zugegeben, die Kolben mit sterilen Schliffstopfen verschlossen und weiter inkubiert. 24 Stunden nach Zugabe des 2-Octanol wird je 1 ml Probe entnommen, mit 2 ml Hexan extrahiert und gaschromatographisch analysiert. Es wurden 37,2 % (Flächen prozent) 2-Octanon und 46,1 % 2-Octanol gefunden.

### Beispiel 6 - Gewinnung von 2-Nonanon aus 2-Nonanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 100 µl 2-Nonanol zugegeben, die Kolben mit sterilen Schliffstopfen verschlossen und weiter inkubiert. 48 Stunden nach Zugabe des 2-Nonanol wird je 1 ml Probe entnommen, mit 2 ml Hexan extrahiert und gaschromatographisch analysiert. Es wurden 32 % (Flächen - prozent) 2-Nonanon und 48,8 % 2-Nonanol gefunden.

### Beispiel 7 - Gewinnung von 3-Octanon aus 3-Octanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden für 20 Stunden auf der rotierenden Schüttelmaschine bei 25 °C und 100 Upm inkubiert. Während dieser Wachstumsphase sind die Kolben mit Wattestopfen steril verschlossen.

Anschließend wird den Kolben je 100 µl 3-Octanol zugegeben, die Kolben mit sterilen Schliffstopfen verschlossen und weiter inkubiert. 72 Stunden nach Zugabe des 3-Octanol wird je 1 ml Probe entnommen, mit 2 ml Hexan extrahiert und gaschromatographisch analysiert. Nach 72 Stunden wurden 4,4 % (Flächen prozent) 3-Octanon und 81,5 % 3-Octanol gefunden.

### Beispiel 8 - Gewinnung von 3-Hexanon aus 3-Hexanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 200 µl 3-Hexanol zugegeben, die Kolben mit sterilen Schliffstopfen verschlossen und weiter inkubiert. 24 und 54 Stunden nach Zugabe des 3-Hexanol wird je 1 ml Probe entnommen, mit 2 ml Hexan extrahiert und gaschromatographisch analysiert. Nach 24 Stunden wurden 70,7 % (Flächenprozent) 3-Hexanon und 24,5 % 3-Hexanol gefunden. Nach 54 Stunden liegt der Gehalt an 3-Hexanon bei über 88 %, während von dem Substrat nur noch 7,4 % vorhanden sind.

### Beispiel 9 - Gewinnung von 1-Hexen-3-on aus 1-Hexen-3-ol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 200 µl 1-Hexen-3-ol zugegeben, die Kolben mit sterilen Schliffstopfen verschlossen und weiter inkubiert. 8 Stunden nach Zugabe des 2-Hexen-2-ol wird je 1 ml Probe entnommen, mit 2 ml Hexan extrahiert und gaschromatographisch analysiert. Es wurden 25,4 % (Flächenprozent) 1-Hexen-3-on und 69,7 % 1-Hexen-3-ol gefunden.

### Beispiel 10 - Gewinnung von 3-Heptanon aus 3-Heptanol

Zwei 100 ml Erlenmeyerkolben mit Schliff mit je 20 ml sterilem Kultivierungsmedium, bestehend aus 0,25 g D-Mannit und 0,25 g Hefeextrakt bei pH 5,6, werden mit 200µl einer Vorkultur von Gluconobacter sp. DSM 12884 angeimpft.

Die Kolben werden 20 Stunden lang auf einer rotierenden Schüttelmaschine bei 25 °C und 100 Upm inkubiert. Während dieser Wachstumsphase sind die Kolben mit Wattestopfen steril verschlossen.

Anschließend wird den Kolben je 200 µl 3-Heptanol zugegeben, die Kolben mit sterilen Schliffstopfen verschlossen und weiter inkubiert. 8 Stunden nach Zugabe des 3-Heptanol wird je 1 ml Probe entnommen, mit 2 ml Hexan extrahiert und gaschromatographisch analysiert. Es wurden 2,3 % (Flächen prozent) 3-Heptanon und 92,2 % 3-Heptanol gefunden.

## Patentansprüche

1. Verfahren zum Umsetzen eines einwertigen sekundären Alkohols mit 5 oder mehr Kohlenstoffatomen zu dem entsprechenden Keton, umfassend die Oxidation des Alkohols zum Keton mit Hilfe eines Bakteriums der Gattung Gluconobacter in einem Fermentationsmedium, **dadurch gekennzeichnet, dass** in der Fermentation 2-Pentanol zu Pentan-2-on, 2-Heptanol zu Heptan-2-on, 1-Penten-3-ol zu 1-Penten-3-on, 1-Hexen-3-ol zu 1-Hexen-3-on, 3-Hexanol zu Hexan-3-on und/oder 3-Heptanol zu Heptan-3-on umgesetzt wird.

2. Verfahren nach Anspruch 1, **dadurch gekennzeichnet, dass** die Umsetzung mit Hilfe eines Bakteriums des Stammes Gluconobacter sp. DSM 12884 bewirkt wird.

3. Verfahren nach einem der vorherigen Ansprüche, **dadurch gekennzeichnet, dass** das Fermentationsmedium Mannit, Malzextrakt, Hefeextrakt, Sojamehl, Baumwollsaatmehl, Weizengluten, Casein, Caseinhydrolysat, Maisquellwasser, Zitronensäure, Essigsäure oder Mischungen oder mehrerer dieser Bestandteile enthält und bei Beginn des Fermentierens einen pH-Wert von 4 bis 8 hat.

4. Verfahren nach einem der vorherigen Ansprüche, **dadurch gekennzeichnet, dass** das zum Fermentieren verwendete Bakterium vor dem Fermentieren in einem Kultivierungsmedium vorkultiviert wird, das Mannit, Malzextrakt, Hefeextrakt, Sojamehl, Baumwollsaatmehl, Weizengluten, Casein, Caseinhydrolysat, Maisquellwasser, Zitronensäure, Essigsäure oder Mischungen zweier oder mehrerer dieser Bestandteile enthält und bei Beginn des Vorkultivierens einen pH-Wert von 4 bis 8 hat.

5. Verfahren nach einem der vorherigen Ansprüche, **dadurch gekennzeichnet, dass** das Fermentieren bei einer Temperatur von 20 bis 40 °C erfolgt,

6. Verfahren nach einem der vorherigen Ansprüche, **dadurch gekennzeichnet, dass** die Gelöstsauerstoffkonzentration im Fermentationsmedium kleiner oder gleich 5 % ist.

7. Verwendung von Gluconobacter sp. DSM 12884 zum Fermentieren eines einwertigen sekundären Alkohols mit 5 oder mehr Kohlensfioffatomen zum entsprechenden Keton.

## Claims

1. Process for converting a monohydric, secondary alcohol having 5 or more carbon atoms into the corresponding ketone, comprising oxidation of the alcohol to form the ketone with the aid of a bacterium of the genus Gluconobacter in a fermentation medium, **characterised in that** in the fermentation, 2-pentanol is converted into pentane-2-one, 2-heptanol is converted into heptane-2-one, 1-pentene-3-ol is converted into 1-pentene-3-one, 1-hexene-3-ol is converted into 1-hexene-3-one, 3-hexanol is converted into hexane-3-one and/or 3-heptanol is converted into heptane-3-one.

2. Process according to Claim 1, **characterised in that** the conversion is carried out with the aid of a bacterium of the strain Gluconobacter sp. DSM 12884.

3. Process according to any one of the preceding claims, **characterised in that** the fermentation medium contains mannitol, malt extract, yeast extract, soya flour, cottonseed flour, wheat gluten, casein, casein hydrolysate, maize steep liquor, citric acid, acetic acid or mixtures or several of these constituents, and has a pH of from 4 to 8 at the start of the fermentation.

4. Process according to any one of the preceding claims, **characterised in that** the bacterium used for the fermentation is pre-cultured before the fermentation in a culture medium which contains mannitol, malt extract, yeast extract, soya flour, cottonseed flour, wheat gluten, casein, casein hydrolysate, maize steep liquor, citric acid, acetic acid or mixtures of two or more of these constituents, and has a pH of 4 to 8 at the start of the pre-culturing.

5. Process according to any one of the preceding claims, **characterised in that** the fermentation is carried out at a temperature of from 20 to 40°C.

6. Process according to any one of the preceding claims, **characterised in that** the dissolved oxygen concentration in the fermentation medium is less than or equal to 5 %.

7. Use of Gluconobacter sp. DSM 12884 for fermenting a monohydric secondary alcohol having 5 or more carbon atoms to form the corresponding ketone.

## Revendications

1. Procédé pour convertir un monoalcool secondaire ayant 5 atomes de carbone ou plus en la cétone correspondante, comprenant l'oxydation de l'alcool en la cétone à l'aide d'une bactérie du genre Gluconobacter dans un milieu de fermentation, **caractérisé en ce que**, dans la fermentation, le 2-pentanol est converti en pentan-2-one, le 2-heptanol est converti en heptan-2-one, le 1-pentén-3-ol est converti en 1-pentén-3-one, le 1-hexén-3-ol est converti en 1-hexén-3-one, le 3-hexanol est converti en hexan-3-one et/ou le 3-heptanol est converti en heptan-3-one.

2. Procédé selon la revendication 1 **caractérisé en ce que** la conversion est accomplie à l'aide d'une bactérie de la souche Gluconobacter sp. DSM 12884.

3. Procédé selon l'une des revendications précédentes **caractérisé en ce que** le milieu de fermentation contient du mannitol, de l'extrait de malt, de l'extrait de levure, de la farine de soja, de la farine de graines de coton, du gluten de blé, de la caséine, un hydrolysat de caséine, de l'eau de trempage du maïs, de l'acide citrique, de l'acide acétique ou des mélanges ou plusieurs de ces constituants et a au début de la fermentation un pH de 4 à 8.

4. Procédé selon l'une des revendications précédentes **caractérisé en ce que** la bactérie utilisée pour la fermentation est pré-cultivée avant la fermentation dans un milieu de culture qui contient du mannitol, de l'extrait de malt, de l'extrait de levure, de la farine de soja, de la farine de graines de coton, du gluten de blé, de la caséine, un hydrolysat de caséine, de l'eau de trempage du maïs, de l'acide citrique, de l'acide acétique ou des mélanges de deux ou plusieurs de ces constituants et a au début de la pré-culture un pH de 4 à 8.

5. Procédé selon l'une des revendications précédentes **caractérisé en ce que** la fermentation a lieu à une température de 20 à 40°C.

6. Procédé selon l'une des revendications précédentes **caractérisé en ce que** la concentration d'oxygène dissous dans le milieu de fermentation est inférieure ou égale à 5 %.

7. Utilisation de Gluconobacter sp. DSM 12884 pour la fermentation d'un monoalcool secondaire ayant 5 atomes de carbone ou plus en la cétone correspondante.
